# FASCICULE DE BREVET EUROPEEN

(11) **EP 0 720 660 B1**
(45) Date de publication et mention de la délivrance du brevet: **13.02.2002**
(21) Numéro de dépôt: 95920951.1
(22) Date de dépôt: 18.05.1995
(51) Int. Cl.: C12Q 1/68, C12Q 1/70

(54) **PROCEDE D'AMPLIFICATION ENZYMATIQUE $i(IN VITRO) D'UN FRAGMENT D'ADN A L'AIDE D'AMORCES EN ESCALIER**
ENZYMATISCHE VERVIELFAELTIGUNG EINES DNA-FRAGMENTS IN VITRO MITTELS EINEM TREPPENFORMIGEN ANFANGSSTUECK
METHOD OF $i(IN VITRO) POLYMERASE CHAIN REACTION OF A DNA FRAGMENT WITH STAIR PRIMERS

(30) Priorité: 18.05.1994 FR 9406055
(43) Date de publication de la demande: 10.07.1996
(73) Titulaire: ARGENE, 09120 Varilhes (FR)
(72) Inventeur: COLIMON, Ronald, F-35000 Rennes (FR)
(74) Mandataire: Warcoin, Jacques
(86) Numéro de dépôt international: FR9500648
(87) Numéro de publication internationale: WO9531568

(56) Documents cités:
- EP-A- 0 393 743
- EP-A- 0 417 842
- EP-A- 0 497 527
- THE LANCET, vol. 11, no. 8610, 3 Septembre 1988 THE LANCET LTD., LONDON, GB;, pages 538-540, F. LAURE ET AL. 'Detection of HIV1 DNA in infants and children by means of the polymerase chain reaction'
- DATABASE WPI Week 9302 Derwent Publications Ltd., London, GB; AN 93-018128 & WO,A,92 22641 (VIRGOGENETICS CORP) , 23 Décembre 1992
- AIDS, vol. 5, no. 8, Août 1991 CURRENT SCIENCE LTD.,LONDON,UK;, pages 1003-1007, D. CANDOTTI ET AL. 'Genetic variability affects the detection of HIV by polymerase chain reaction' cité dans la demande
- ANALYTICAL BIOCHEMISTRY, vol. 186, no. 1, Avril 1990 ACADEMIC PRESS, INC., NEW YORK, US;, pages 64-68, G. SARKAR ET AL. 'Characterization of polymerase chain reaction amplification of specific alleles'

## Description

La présente invention concerne un procédé d'amplification enzymatique d'un fragment d'ADN in vitro.

La présente invention concerne également des procédés de diagnostic basés sur la détection d'ADN et dans lesquels on utilise des méthodes d'amplification d'ADN, en particulier la détection des séquences de virus tels que le virus de l'immunodéficience humaine VIH.

La présente invention concerne également des kits d'amplification d'ADN ou kits de diagnostic pour la mise en oeuvre des procédés selon l'invention.

Plus particulièrement, la présente invention se rapporte aux méthodes d'amplification enzymatique in vitro de séquences d'acide nucléique ADN monobrin ou double brin impliquant l'utilisation d'amorces, notamment la méthode dite "*PCR*".

Les techniques d'amplification enzymatique d'ADN sont bien connues de l'homme de l'art. Les techniques d'amplification in vitro, notamment par PCR, ont été décrites dans la littérature et leur mise en oeuvre et perfectionnements ont fait l'objet de demandes de brevet. On peut citer en particulier les publications européennes EP 201 184 et EP 200 362 sur la technique de base pour la méthode PCR.

Les techniques d'amplification génique in vitro, notamment la méthode dite *"PCR"* (Polymerase Chain Reaction) (21), connaissent un développement rapide en microbiologie. En virologie, leur grande sensibilité en fait un outil de choix pour le diagnostic et les études épidémiologiques à côté des techniques d'isolement et de sérologie.

Plus particulièrement, la présente invention concerne un perfectionnement de ces méthodes d'amplification, pour améliorer l'amorçage et permettant d'amplifier une séquence génomique comportant des mutations dans la région des amorces.

Dans ces méthodes d'amplification enzymatique d'ADN, l'amplification de la séquence s'effectue par cycles successifs. Un cycle comporte plusieurs étapes : une première étape de dénaturation thermique de l'ADN qui sépare, le cas échéant, l'ADN double brin en deux mono-brins, une seconde étape d'hybridation des amorces sur les séquences des mono-brins qui leur sont complémentaires, et une troisième étape d'élongation à partir des extrémités 3' des amorces à l'aide d'un ADN polymérase. On utilise des amorces oligonucléotidiques spécifiques qui vont s'hybrider sur des séquences qui leur sont complémentaires encadrant à ses extrémités 5'P le fragment d'ADN à amplifier. Les extrémités 3'OH de ces amorces constituent le point de démarrage d'une élongation effectuée, dans le sens 5' → 3', par les enzymes de réplication de l'ADN appelées *"ADN polymérases*".

Pour une amplification, le milieu réactionnel contient donc l'ADN à amplifier, les amorces, l'ADN polymérase, ainsi que d'autres éléments tels qu'un tampon, des sels, des désoxynucléotides tri-phosphates. Chaque brin du fragment sert alors de matrice à l'enzyme qui synthétise les brins complémentaires ; le facteur de multiplication est alors de deux. Les oligonucléotides "amorces" (primer) sont à nouveau remis à hybrider avec les brins d'ADN provenant du premier cycle d'amplification, chaque brin servant de matrice à l'ADN polymérase. Le facteur d'amplification sera alors de quatre - et ainsi de suite - avec doublement théorique des copies du fragment d'ADN à chaque cycle.

Dans certaines techniques d'amplification enzymatique d'ADN telles que la technique PCR, les produits issus de ce premier cycle, puis de chacun des cycles successifs, sont dénaturés par la chaleur. L'emploi d'une polymérase thermostable, la Taq polymérase, a permis de développer des cycleurs automatiques (hybridation/polymérisation enzymatique/ dénaturation thermique), rendant plus aisé l'emploi routinier de la méthode. Les conditions opératoires des différentes étapes se distinguent en effet essentiellement par la température à laquelle elles ont lieu. les conditions de dénaturation correspondent généralement à une élévation de la température du milieu réactionnel au-dessus de 90°C, l'hybridation a lieu généralement entre 50 et 70°C et l'élongation par l'ADN polymérase peut s'effectuer à des températures relativement élevées, de l'ordre de 70°C si l'on utilise une ADN polymérase stable à la chaleur.

En revanche, dans d'autres techniques d'amplification enzymatique d'ADN, les produits issus du premier cycle, comme de chacun des cycles successifs, ne sont pas dénaturés. Il s'agit de méthode isotherme.

Dans les méthodes amplification enzymatique d'ADN in vitro, les amorces sont toujours choisies, de préférence, dans les régions génomiques les plus conservées (18).

De nombreux problèmes doivent être résolus pour faire d'une méthode d'amplification enzymatique d'ADN in vitro, notamment de la PCR, un outil fiable de diagnostic, compte tenu de problèmes de contamination, reproductibilité et quantification (3, 6, 11, 16). Le risque d'une amplification et d'une détection faussement négative pour cause de mutation est également un inconvénient majeur de ces méthodes. Ce risque est plus important pour les virus à ARN que pour les virus à ADN.

En effet, les variations génomiques fréquentes, notamment chez les virus à ARN [virus de l'immunodéficience humaine (VIH), virus de l'hépatite C, picornavirus, etc...] peuvent entraîner l'absence d'amplification et entravent la mise au point de diagnostics fiables par ces méthodes (4). A cause de mutations dans le fragment d'ADN à amplifier, correspondant à la région complémentaire de l'extrémité 3' d'une amorce, le rendement de la méthode d'amplification par PCR peut se trouver altéré.

Plusieurs solutions ont été préconisées dans l'état de la technique pour résoudre ce problème.

Selon une première approche, on a proposé d'utiliser plusieurs couples d'amorces, encadrant des régions différentes du génome, testés en parallèle sur le même échantillon. Ceci impose la multiplication des réactions d'amplification et la multiplication des sondes d'hybridation, ou encore la mise en oeuvre de PCR secondaires visant à améliorer l'amplification d'échantillons contenant une faible quantité d'ADN (*nested-PCR*). Il a été aussi proposé d'associer différents couples d'amorces, mais au sein de la même réaction ("*multiplex*") (5, 24). La sensibilité, dans ce dernier cas, peut encore être diminuée par compétition entre les diverses amplifications dans le même tube. EP-A-0 393 743 décrit l'utilisation d'amorces "en escalier" dont la plus courte est en concentration majoritaire. Une autre approche a consisté à étudier des modifications de l'extrémité 3' de l'amorce. Certains auteurs préconisent en effet d'insérer une double désoxythymidine en 3', car quels que soient les nucléotides de la cible, ce type de non-appariement portant sur les deux bases de la terminaison 3', assure un bon rendement d'amorçage. Les amorces substituées avec une 3' inosine (amorces dégénérées) ont été également préconisées (16, 17).

Le but de la présente invention est de fournir un procédé d'amplification enzymatique in vitro d'ADN comportant une méthode d'amorçage simple et surtout fiable permettant, au cours d'une seule réaction d'amplification, d'amplifier une région génomique donnée, que la région amorcée comporte ou non des variations nucléotidiques. Un autre but de la présente invention est que le procédé d'amplification aboutissant néanmoins à la synthèse d'une seule bande détectée sous la forme d'un signal unique à l'électrophorèse et en southern-blot nécessitant l'utilisation d'une seule sonde d'hybridation.

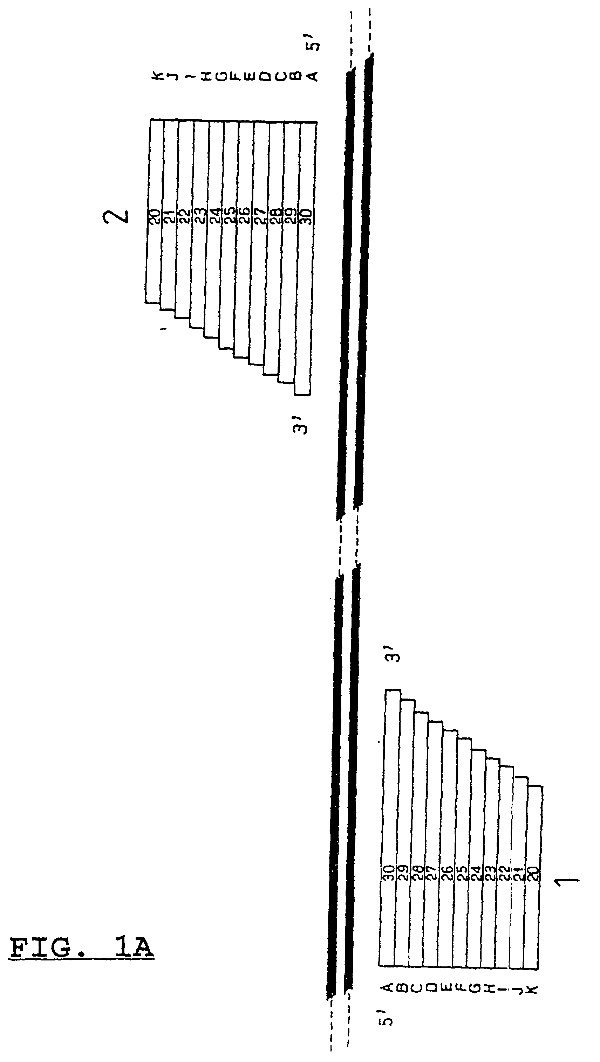

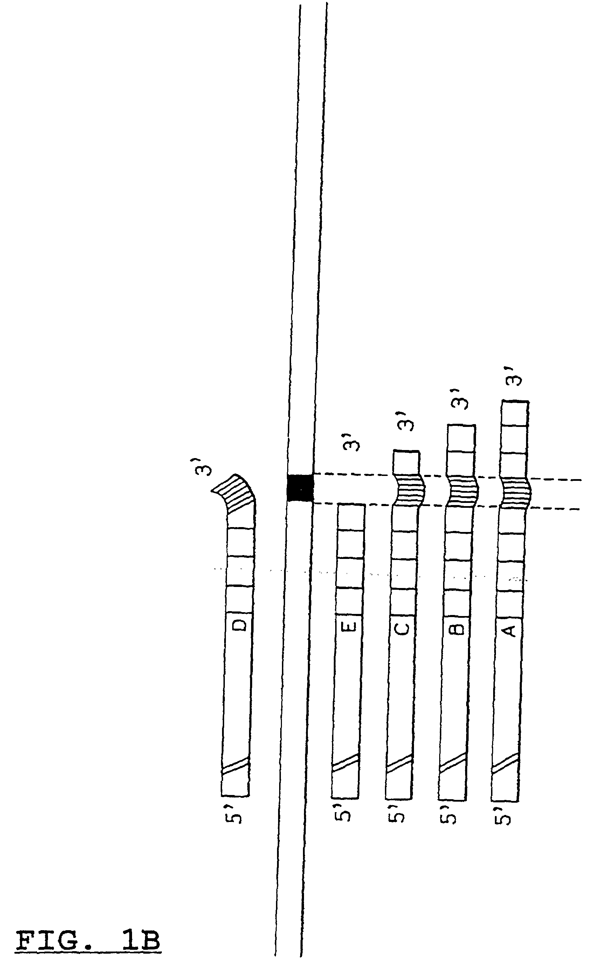

La présente invention utilise des amorces dites *"en escalier"* (*"stair-primers"*). L'amorce classique est remplacée par un mélange (*"set"*) d'oligonucléotides de longueur variable, l'extrémité 5' étant fixe, de sorte que la longueur du fragment amplifiée est constante, et l'extrémité 3' de ces oligonucléotides se décalant l'une par rapport à l'autre, par exemple base par base, comme illustré à la Figure 1. S'il existe une mutation ponctuelle, voire une délétion ou une insertion, l'amorçage, impossible avec l'oligonucléotide dont l'extrémité 3' correspond à la mutation, délétion ou insertion, pourra être assuré par des oligonucléotides dont l'extrémité 3' se situe en amont et/ou en aval de cette mutation.

Il convient de relever que le problème d'amorçage ne se présente qu'au premier cycle de l'amplification, puisque la mutation est "corrigée" par la séquence de l'amorce. Dès le second cycle, pratiquement, toutes les espèces moléculaires du mélange peuvent initier l'amplification. L'empilement des séquences des oligonucléotides constituant chacun des deux mélanges prenant l'allure de marches d'escalier a inspiré le nom de "amorces en escalier" (*"stair-primers"*).

Selon l'invention, il est nécessaire, pour la meilleure efficacité des "amorces en escalier", notamment pour une bonne compatibilité des amorces au cycle suivant, que les séquences des oligonucléotides du mélange dérivent d'une même séquence et non pas d'un mélange de séquences différentes. Il est aussi nécessaire que les différentes molécules du mélange soient en quantité équimolaires. Il faut éviter qu'une espèce moléculaire du mélange, incapable d'amorcer l'amplification, mais plus abondante, ne bloque l'amplification par compétition.

La présente invention a donc pour objet un procédé d'amplification enzymatique in vitro d'un fragment d'ADN dans lequel on utilise deux amorces respectivement amont et aval dont au moins une est une amorce dite *"en escalier"* qui consiste en un mélange d'oligonucléotides, les oligonucléotides constituant le mélange étant en quantités équimolaires, de tailles différentes, de séquences complémentaires à la même région en amont ou en aval du dit fragment à amplifier et chaque oligonucléotide ayant, à partir de son extrémité 5', dans le sens 5'→3', la même séquence que les oligonucléotides plus petits du mélange.

De préférence, on utilise deux amorces en escalier respectivement amont et aval qui consistent chacune en un mélange d'oligonucléotides, les oligonucléotides constituant chaque mélange amont ou aval étant en quantités équimolaires, de tailles différentes, de séquences complémentaires à la même région respectivement en amont ou en aval du dit fragment à amplifier et chaque oligonucléotide ayant, à partir de son extrémité 5', dans le sens 5'→3', la même séquence que les oligonucléotides plus petits du même mélange.

Conformément à cette définition, les oligonucléotides de chaque mélange ont donc tous la même extrémité 5' et ne se distinguent qu'à l'extrémité 3'.

Il convient de remarquer que dans la présente description, on entend par "amorce" une quantité déterminée en oligonucléotide. C'est pourquoi on parle d'amorce en escalier, constituée par un mélange d'oligonucléotides", et non d'un "mélange d'amorces". Cette terminologie reflète le fait que la quantité globale des différents oligonucléotides compris dans chaque mélange est la même que la quantité d'oligonucléotides constituant une amorce classique unimoléculaire utilisée dans les méthodes d'amplification classique d'ADN in vitro connues. Pour le reste, le choix des séquences et la taille des oligonucléotides sont régies par les mêmes critères que pour les amorces uni-moléculaires classiques, c'est-à-dire des critères de complémentarité et de spécificité vis-à-vis de la séquence cible à amplifier.

Selon l'invention, dans son principe, le nombre d'oligonucléotides dans chaque mélange, c'est-à-dire de façon imagée, le nombre de "marches d'escalier", n'est pas limitatif. Plus ce nombre est grand, plus la fiabilité du procédé sera grande.

Si l'on considère qu'au-delà de deux bases à partir de l'extrémité 3', les mutations n'interviennent pas, ou de manière non significative, c'est-à-dire n'affectent pas l'efficacité d'amorçage et qu'en outre les mutations ne portent jamais sur plus de deux bases consécutives, cinq oligonucléotides pourront suffire dans chaque mélange-amorce. Si l'on augmente ces nombres de bases à trois, sept oligonucléotides pourront alors suffire.

En pratique, un mélange de cinq à quinze oligonucléotides convient, en particulier, lorsque la région cible de l'amorce peut subir des mutations et/ou des délétions ou insertions de séquences sur un à cinq nucléotides et que la différence de taille entre chaque oligonucléotides et celui immédiatement plus grand et plus petit est de un ou deux nucléotide(s).

En pratique, une mutation étendue du génome à amplifier concernant simultanément plusieurs nucléotides en 3' des molécules composant le mélange est statistiquement très improbable. En particulier, on considère en pratique que les mutations ne portent jamais sur plus de trois bases consécutives. En outre, on considère qu'au-delà de trois bases de l'extrémité 3', les mutations n'interviennent plus dans le rendement de l'amplification.

Dans un mode de réalisation de l'invention, la différence de taille entre chaque oligonucléotide et celui immédiatement plus grand ou plus petit, c'est-à-dire de façon imagée la "largeur des marches" n'est pas nécessairement de 1 nucléotide comme on l'a vu et peut être de 1 à 4 nucléotides, de préférence 1 à 3. En effet, il peut être avantageux, dans certains cas, d'avoir des marches de plusieurs nucléotides, par exemple de deux à quatre. L'augmentation de la largeur des marches peut permettre, dans certains cas, de diminuer le nombre d'oligonucléotides, c'est-à-dire le nombre de marches, et augmenter ce faisant la fiabilité du système sans avoir recours à un nombre excessif d'oligonucléotides, en particulier dans les cas où l'on sait que la région variable s'étend sur un assez large domaine, ou lorsque la variabilité est due à des mutations ponctuelles mais fréquentes. De même, la "largeur des marches" n'est pas nécessairement identique entre les différents oligonucléotides d'une même amorce escalier, c'est-à-dire d'un mème mélange.

Lorsqu'on utilise deux amorces en escalier, le nombre de marches, c'est-à-dire le nombre d'oligonucléotides, n'est pas nécessairement identique dans le mélange d'amorce amont et le mélange d'amorce aval. Si l'on estime que la région aval ou amont de la cible est plus conservé, il est possible d'envisager un nombre réduit de marches d'oligonucléotides dans l'amorce aval ou amont respectivement, à condition que la quantité molaire globale d'oligonucléo-tides reste identique dans chaque mélange.

De même, la symétrie des deux mélanges n'est pas obligatoire en ce qui concerne la taille des différents nucléotides et la largeur des marches.

Toutefois, pour des raisons d'homogénéité quant à la stabilité et à la spécificité de l'hybridation, il peut être préférable d'utiliser des amorces amont et aval qui soient symétriques en termes de nombre d'oligonucléotides et de différence de taille entre eux, c'est-à-dire de largeur de marches. Donc, dans un mode de réalisation de la présente invention, le nombre et les tailles des oligonucléotides respectifs dans les amorces amont et aval sont identiques. Dans ce mode de réalisation particulier, les amorces amont et aval comportent des oligonucléotides de tailles identiques deux à deux.

De préférence encore, dans une amorce en escalier selon l'invention ou dans chaque amorce, les différences de tailles entre chaque oligonucléotide, l'oligonucléotide immédiatement plus grand ou immédiatement plus petit, sont identiques

L'avantage principal du procédé selon l'invention est de réduire le risque d'amplification et de détection faussement négative, et même de l'éliminer complètement, si l'on s'adresse à une région du génome qui ne subit pas de modifications importantes du type délétion ou insertion supérieure à cinq nucléotides dans la région des amorces, en particulier, dans le cas le plus fréquent, où la région des amorces ne comporte que des mutations ponctuelles.

De préférence, selon l'invention, les amorces en escalier sont en effet constituées d'oligonucléotides complémentaires aux régions les mieux conservées des génomes constituant le fragment, de préférence avec des régions qui ne subissent que des mutations ponctuelles.

En outre les amorces en escalier selon l'invention permettent dans tous les cas de régulariser le rendement des amorces, ce qui est très important lorsqu'on met en oeuvre une méthode d'amplification quantitative.

Un autre avantage de la méthode selon l'invention est de renforcer la spécificité des amorces. En effet, dans la mesure où la quantité de chaque oligonucléotide par rapport à la quantité d'amorces unimoléculaires dans les méthodes classiques, est divisée par le nombre d'oligonucléotides dans le mélange, l'hybridation non spécifique diminue d'autant. Par ailleurs, les mélanges d'oligonucléotides selon l'invention sont amenés nécessairement à avoir des amorces comportant des oligonucléotides de taille plus grande. Cette dernière caractéristique renforce également la spécificité. Les amorces escalier selon l'invention constituent donc un moyen supplémentaire d'éviter les amorçages non spécifiques. Le fait que chaque espèce moléculaire représente seulement le énième (dans le cas où le mélange comporte n nucléotides) de la molarité nécessaire pour amplifier efficacement explique vraisemblablement le fait qu'on ne voit pas apparaître de bandes non spécifique due à la multiplicité des terminaisons 3' comme on pouvait le craindre.

De préférence, la taille des oligonucléotides dans une amorce en escalier selon l'invention, est comprise de 15 à 40 nucléotides, en particulier de 20 à 30.

Comme on l'a vu, le procédé selon l'invention est applicable dans tout procédé impliquant l'utilisation des amorces d'amplification d'ADN, notamment les procédés de diagnostic impliquant la détection in vitro d'un fragment d'ADN dans lequel on utilise un procédé d'amplification enzymatique in vitro du dit fragment d'ADN selon l'invention.

Le procédé selon l'invention peut en particulier être adapté au diagnostic génétique. Pour les agents pathogènes, la possibilité de séquencer de nombreuses souches permet de choisir les régions conservées et minimise ainsi les inconvénients dûs aux variations génomiques. Cette possibilité est plus restreinte pour un génome humain et le procédé selon l'invention à l'aide d'amorces en escalier permet d'améliorer grandement la fiabilité du diagnostic par amplification d'ADN dans le domaine de la génétique humaine.

Le procédé selon l'invention peut permettre, à partir d'un gène connu dans une espèce déterminée de rechercher le même gène dans une espèce voisine. Le nombre d'oligonucléotides peut être augmenté et l'homologie avec les amorces des séquences recherchées peut être ainsi plus basse.

Les amorces en escalier selon l'invention sont en particulier applicables pour détecter des régions plus stables du VIH que celles exemplifiées ci-après et elles sont également appliquées pour le diagnostic de l'hépatite C, pour la mise au point d'amorces génériques pour certains entérovirus et pour le diagnostic des virus de l'herpès.

Le procédé selon la présente invention, de par sa simplicité, rend des services en améliorant la fiabilité du diagnostic par amplification d'ADN, notamment du type PCR, en particulier pour le VIH.

D'autres avantages et caractéristiques de la présente invention apparaitront à la lumière d'un mode détaillé de réalisation qui va suivre et des figures 1 à 5.

La présente invention a également pour objet un kit de diagnostic pour la mise en oeuvre d'un procédé de diagnostic selon l'invention, caractérisé en ce qu'il comporte des amorces d'amplifications enzymatiques in vitro d'un fragment d'ADN à détecter, les dites amorces consistant en des amorces en escalier telles que définies ci-dessus pour la mise en oeuvre d'un procédé d'amplification selon l'invention.

La **Figure 1** représente le principe des *"amorces eu escalier"*.
A : Exemple d'*"amorces en escalier"*. L'amorce classique est remplacée par un mélange de 11 oligonucléotides de 20 à 30 bases (A à K), possédant une extrémité 5' identique et une extrémité 3' se déplaçant base par base.
B : Amorçage de l'ADN par les amorces en escalier en cas de mutation ponctuelle de la séquence à amplifier : le carré noir figure le nucléotide muté présent dans la séquence cible de l'amorce. L'oligonucléotide D, incapable de s'apparier en 3', est suppléé par les oligonucléotides dont l'extrémité 3' se situe en amont ou en aval du nucléotide muté (amorces A, B, C, E...).

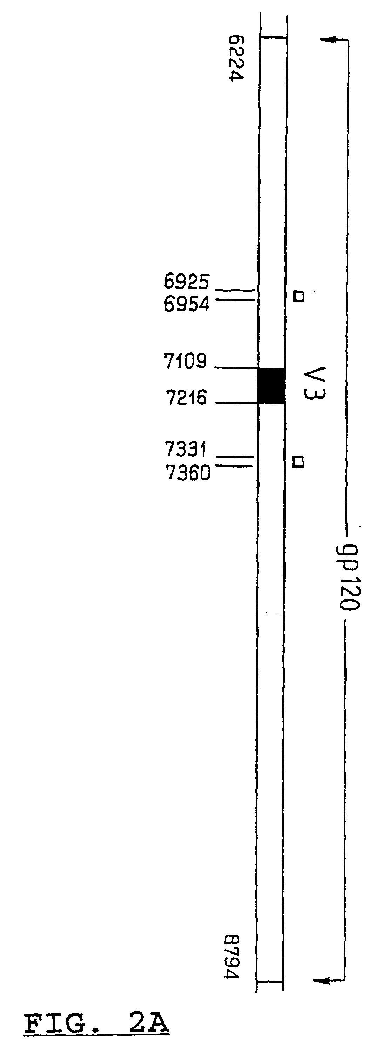

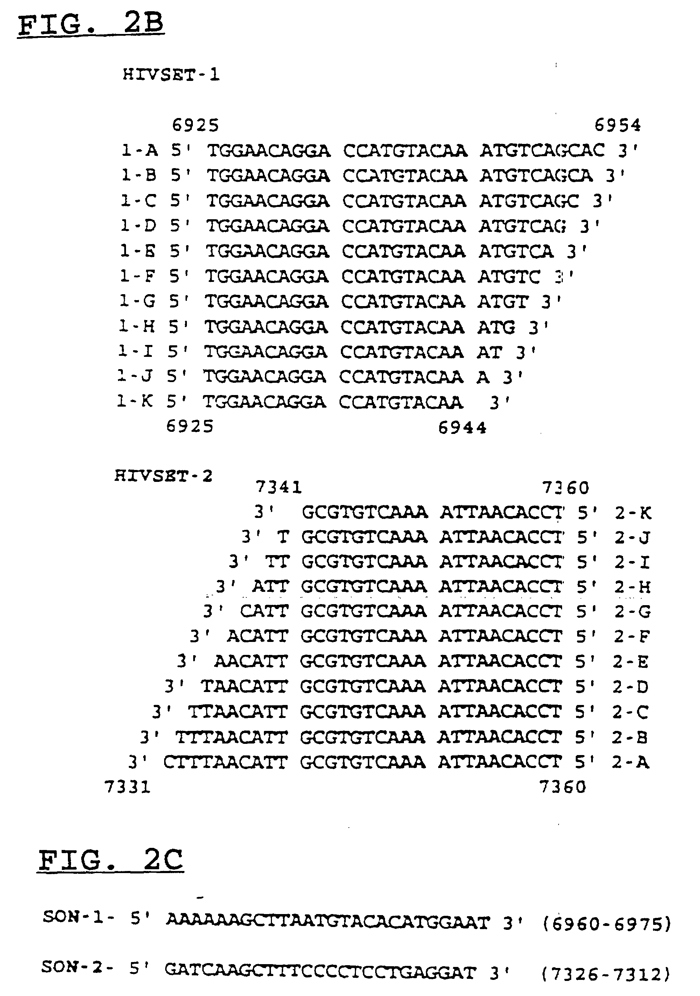

La **Figure 2** représente la position de la région du génome du VIH amplifiée par HIVSET, et séquences des amorces.
A : Situation de la région amplifiée de la gp 120. Le symbole (□) représente la position des amorces encadrant les séquences V3.
B : Séquences des oligonucléotides composant HIVSET1 et HIVSET2.
C : Amorces internes pour la sonde. Ces amorces aboutissent à la synthèse d'une sonde de 368 pb. En 5', un site de restriction Hind III a été rajouté pour permettre le clonage du fragment.

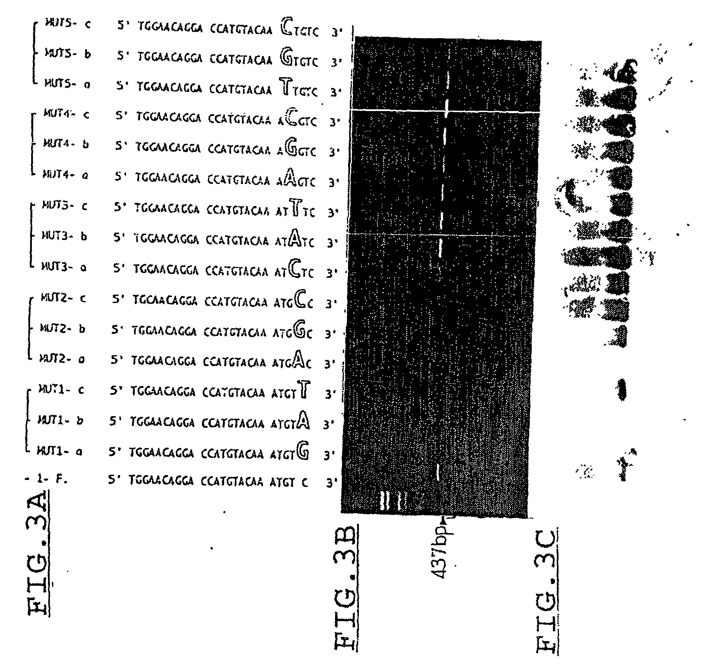

La **Figure 3** représente les résultats des amorces mutées.
A : Séquence des amorces utilisées.
B : Gel d'électrophorèse après amplification par des amorces mutées.
   Marqueur de taille des fragments d'ADN (Boehringer DNA molecular-weight marker VI - Cat. N° 1062590). Amplifcation après 25 cycles (10µ de dépôt) de l'ADN extrait des cellules 8E5 contenant 1 copie de génome de VIH par cellule, par les différentes amorces. L'amorce 1-F est l'amorce sauvage. Les amorces Mut1-a/b/c à Mut5-a/b/c sont les amorces mutées de la position 1 à la position 5 à partir de leur extrémité 3' (a, b, c = substitution par les 3 autres nucléotides).
C : Hybridation avec la sonde marquée à la digUTP.

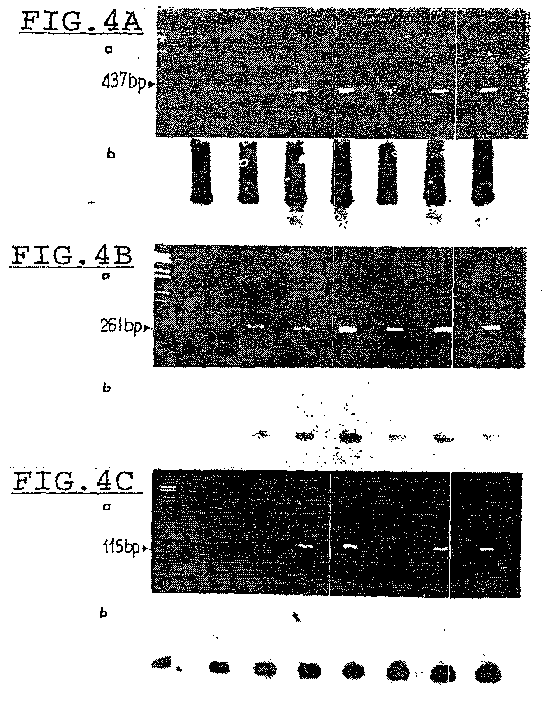

La **Figure 4** représente des exemples d'amplifications des souches de VIH par HIVSET, polV2, et SK38-39.
Amplifications avec A) HIVSET, B) polV2, C) SK38-39 respectivement (a: gel, b: hybridation avec les sondes respectives marquées à la digUTP).
En 1 Marqueur de taille des fragments d'ADN, de 2 à 8 Amplifications de 7 souches de patients avec une sérologie positive pour le VIH. La souche en 2 est celle qui n'est pas amplifiée par polV2.

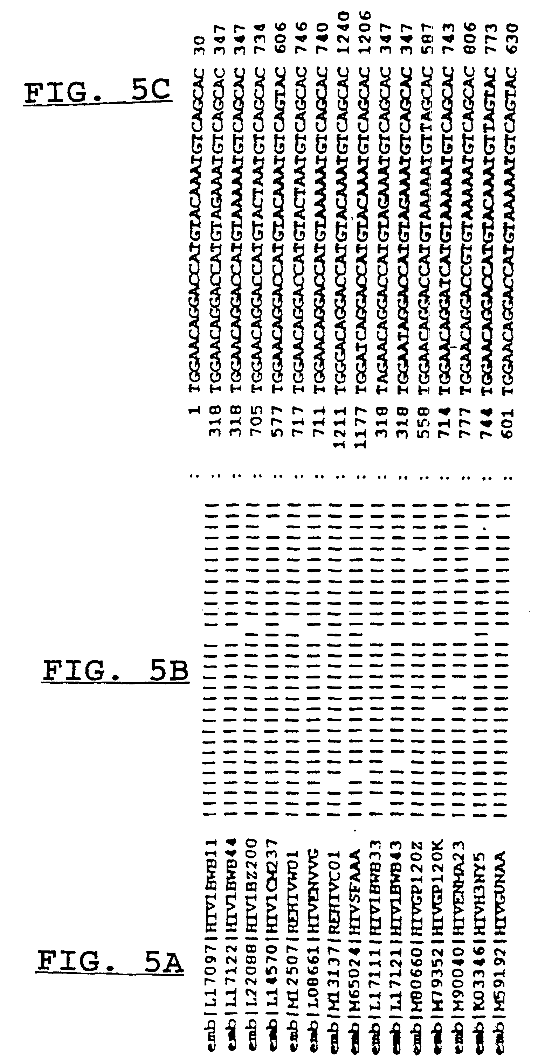

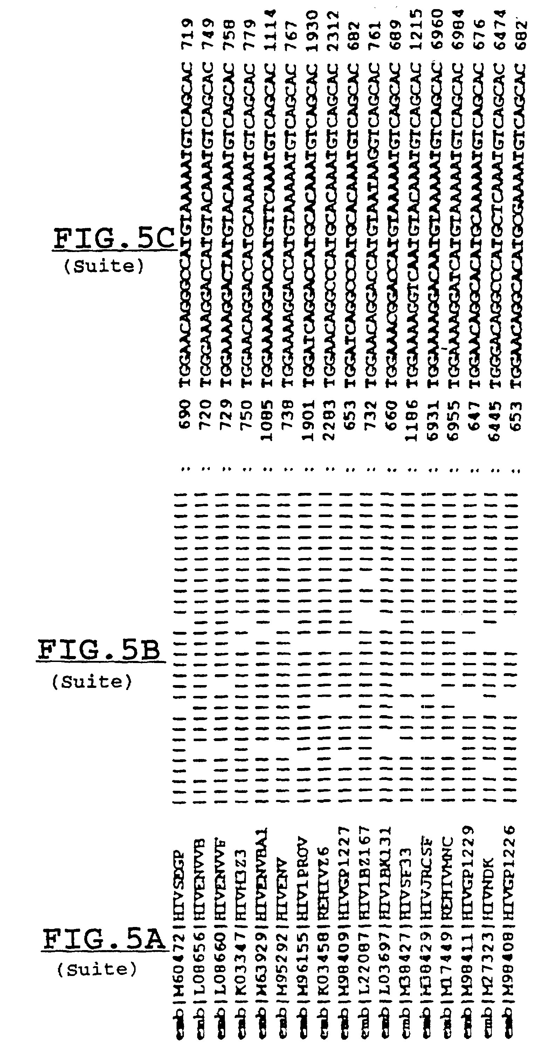

La **Figure 5** représente des résultats de l'alignement par le programme Blastn.
A : Référence des séquences dans la banque Embl.
B : Représentation schématique des homologies de bases entre l'amorce 1-A et la région génomique homologue.
   Les 32 séquences représentent l'ensemble des mutations observées parmi 210 séquences. Les mutations sont symbolisées par l'absence du trait vertical correspondant à la position de la base mutée. Quel que soit le génome, plus de la moitié des espèces moléculaires du mélange sont théoriquement capables d'amorcer l'amplification au premier cycle.
C : Séquence génomique homologue de l'amorce 1-A.

On a appliqué le procédé selon l'invention à la détection du VIH, dont la variabilité génomique a été largement étudiée (Revues 15, 23). Chaque isolat peut être considéré comme une population de génomes uniques très proches (quasi-espèces) (19). Des amorces selon l'invention, ("stair-primers") ont été volontairement sélectionnées dans les séquences génomiques du VIH les plus variables, celles codant pour la glycoprotéine gp 120, dans les régions encadrant la boucle V3. On les a testées sur des souches isolées de patients séropositifs ainsi que sur des lymphocytes de malades, et comparé les résultats à ceux obtenus avec des amorces classiques sélectionnées dans les régions les plus stables du génome (gènes pol et gag).

### 1) Matériel et méthodes :

L'amplification a porté sur un fragment de 437 pb (nucléotides 6925 à 7361 de la souche HXB2, Embl Accession n° K03455), dans la région codant pour la glycoprotéine gp 120. Ce fragment est centré par les séquences de la boucle V3.
**1.1) Amorces "mutées" :** on a d'abord étudié l'influence, sur le rendement de l'amplification, de mutations génomiques de l'extrémité 3' des amorces choisies, dans les conditions de la réaction. Les amplifications, du même ADN proviral du VIH, ont été faites avec des amorces dérivées de l'amorce 1-F (Figure 2), comportant des mutations ponctuelles des cinq nucléotides de l'extrémité 3'. Une série de 16 amorces de 25 nucléotides a été synthétisée (Laboratoire de Biochimie de la Faculté de Médecine de Rennes- Pr. Legall). Chacun des 5 nucléotides à muter a été remplacé par les 3 autres nucléotides (Voir Résultats, Figure 3).
   L'ADN amplifié est celui de la gp 160 de la souche de VIH Bru (virus recombinant VVTG 1139). Seule l'amorce d'amont est mutée, l'amorce d'aval est inchangée. Les étapes de l'amplification sont celles décrites plus bas.
**1.2) Choix des oligonucléotides composant les amorces en escalier:** L'amorce classique est remplacée par un mélange d'oligonucléotides de longueur variable, l'extrémité 5' étant fixe (longueur du fragment amplifié constante), l'extrémité 3' se décalant base par base (figure 1).
   On a réalisé pour chaque amorce un mélange de 11 oligonucléotides de 20 à 30 bases, 1-A à 1-K pour l'amorce en amont HIVSET1, et 2-A à 2-K pour l'amorce en aval HIVSET2 (Figure 2). Chaque amorce ou mélange est utilisé à la molarité de 1 µM, chaque oligonucléotide intervenant pour 1/11 dans la molarité finale (mélange en quantité égale d'une suspension 1 µM de chacune des 11 oligonucléotides).
**1.3) Amorces classiques :**
   Les amorces polV2 encadrant une région de 261 pb dans le gène pol ont été sélectionnées par le logiciel PCrare (Eurogentec Bruxelles)(10). Les amorces SK38-39 encadrant une région de 115 pb dans le gène gag ont été décrites par C.Y. Ou et coll (20) (Tableau 1). Ces amorces sont utilisées en pratique courante.
   Les séquences SK38-39, SK40-41 et de polV2 A à D sont décrites dans Genbank n° KO2007; les positions sur le génome sont décrites dans le Tableau 1. 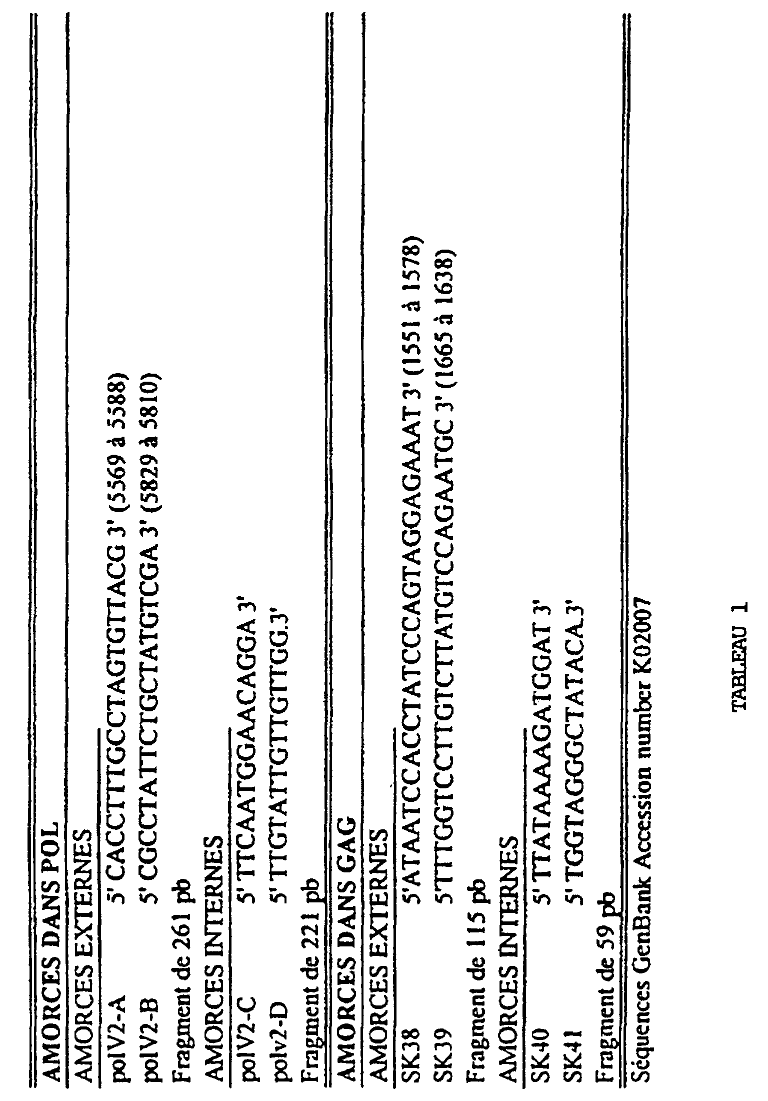
**1.4) Comparaison des séquences amorces HIVSET à celles d'une banque génomique :**
   Pour étudier la position des mutations rencontrées dans les régions des amorces HIVSET, on a recherché par le programme Blastn (1) (Serveur Blast@crihan.fr) l'homologie entre les oligonucléotides HIVSET et les séquences de VIH référencées à ce jour dans la banque Embl (dernière version).
**1.5) Echantillons biologiques :**
   . Souches isolées de malades. Les souches proviennent de la coculture de lymphocytes de 72 patients, avec une sérologie VIH positive, vus au CHR de Rennes. La culture virale est réalisée à partir de la fraction lymphocytaire du sang total, séparée en gradient de Ficoll. Les lymphocytes sont repris en milieu RPMI additionné de sérum foetal de bovidé et d'interleukine-2, et cultivés en présence de lymphocytes de sujets séronégatifs (2X10⁶ cellules/ml) stimulés à la phytohémagglutinine (7). Au bout de 21 jours, après détection de l'antigène p24 (Laboratoires Abbott), la suspension cellulaire est recueillie et stockée à -80° sous forme d'aliquots de 1 ml contenant environ 3X10⁶ de cellules.
   . Le génome du VIH a été recherché directement (avant coculture) dans l'ADN des lymphocytes de 29 de ces patients.
   . Les témoins négatifs sont des lymphocytes de donneurs de sang séronégatifs pour le VIH.
   . ADN proviral de référence. L'ADN de la souche BRU a été utilisé comme cible de référence. Cet ADN provient de 3 sources : a) Cellules 8E5 (1 copie de provirus intégré par cellules)(8). Ces cellules sont entretenues en milieu RPMI additionné de sérum foetal de bovidé. b) ADN de la gp 160 provenant du surnageant de culture sur cellules BHK 21 du virus recombinant de la Vaccine VVTG 1139 (Laboratoire Transgène S.A.-Strasbourg-France-). c) Plasmide pBT1 (souche BRU) fourni gracieusement par le Pr. Montagnier (Institut Pasteur Paris).
**1.6) Extraction de l'ADN proviral des lymphocytes :** Elle est réalisée sur 50 µl de la suspension cellulaire, soit environ 150 000 cellules. La déprotéinisation est faite sous un volume de 500 µl en tampon de lyse (Tris 0,1M - EDTA 0,01M - SDS 0,5 %) contenant 200 µg/ml de protéinase K (BOEHRINGER)(1 heure à 37°). Deux extractions par le phénol-chloroforme sont suivies d'une précipitation en alcool absolu en présence d'acétate de Na (0,3 M final). Les acides nucléiques sont repris dans 70 µl d'eau stérile et stockés à -80°. Les extractions sont faites par groupe de 18 (17 cocultures de lymphocytes - souches - ou culots de lymphocytes de patients séropositifs, et 1 culot de lymphocytes de donneur de sang séronégatif).
**1.7) PCR : Région V3 (HIVSET) :** La réaction est faite sous volume de 50 µl.
   L'échantillon à amplifier (l'équivalent de 40 000 cellules dans 15 µl) est recouvert de 100 µl d'huile de paraffine, puis chauffé à 95° pendant 3 minutes. La température est maintenue en plateau à 70° pendant la distribution des différents réactifs : 3,75 µl de chaque set 10 µM, 5 µl de tampon Taq 10X (Tris Hc10,6 M, pH 8,4, SO₄⁻(NH₄)₂ 0,17 M, B₂ mercapto-éthanol 0,1 M, BSA 1,7 mg/ml, MgCl₂ 1,5 mM), 5 µl de mélange des 4 nucléotides dTTP/dATP/dCTP/dGTP en solution 1 mM (Pharmacia) et 2 unités de Taq polymerase (Perkin Helmer). On réalise 35 cycles d'amplification : 93°C, 30 secondes ; 52°C, 1 minute 20 : 72°C, 1 minute 20 (25 cycles pour les amorces mutées).
   . Région pol (polV2) - La réaction est faite sous volume de 25 µl. L'équivalent de 40 000 cellules est additionné de 2,5 µl de chaque amorce à 10 µM, 2,5 µ de tampon Taq 10 X (KCL 50 mM, MgCl2 1,5 mM, Tris 10 mM pH 8,4, gélatine 1 mg/ml), 2,5 µl des nucléotides à 2mM, 1 UI de Taq polymérase. Après dénaturation à 92°C pendant 10 minutes, on réalise 35 cycles d'amplification : 93°C, 15 secondes : 55°C, 1 minute 15 secondes : 72°C, 1 minute 15 secondes.
   . Région gag SK38-39 - La réaction est faite avec les primers SK38-39 dans les mêmes conditions de volume et de concentration des différents réactifs. Après dénaturation à 92°C pendant 10 minutes, on réalise 35 cycles d'amplification comme suit : 93°C pendant 45 secondes, 55°C pendant 45 secondes, 70°C pendant 40 secondes.

   - **Analyse des produits d'amplification :**
      . Gel : L'analyse est faite par électrophorèse de 10 µl des produits amplifiés, et, pour les amorces mutées : produits amplifiés et dilutions de 10⁻¹ à 10⁻⁴. On utilise un gel d'agarose à 2 % contenant 0,5 µg/ml de bromure d'éthidium, en tampon Tris - Acétate (Tris 0,04M - EDTA 0,001M - pH 7,7 ajusté par l'acide acétique glacial). Après dénaturation du gel (NaOH 0,5 M - NaCI 1,5M) neutralisation (Tris 0,5M - NaCI 0,5 M), l'ADN amplifié est transféré sous vide sur filtre de nylon (Hybond°). La membrane est lavée dans du SSC 2X puis séchée à l'air et fixée 40 secondes par exposition aux UV.
      . Sondes d'hybridation : Les sondes d'hybridation sont obtenues par PCR, à partir des amorces internes (Figure 2C, Tableau 1), et incorporation d'UTPs marqués à la digoxigénine (Boehringer)(9). L'ADN matritiel provient du surnageant de culture sur cellules BHK 21 du virus recombinant WTG 1139, pour le fragment amplifié par HIVSET, et de pBT1 pour les fragments amplifiés par polV2 et par SK38-39.
      . Hybridation des membranes : Les membranes sont hybridées 16 heures avec les sondes spécifiques dans 10 ml de tampon d'hybridation (SSC 5X, réactif bloquant 0,5 %, sarcosinate de sodium 0,1 %, SDS 0,02 %) à 60° pour HIVSET et polV2 (50°C pour SK38-39), et 20 ng de sonde dénaturée par ébullition pendant 10 minutes sont ajoutés. Après hybridation, les filtres sont lavés 2 fois 5 minutes dans du SSC 2X-SDS 0,1 %, puis deux fois 15 minutes dans du SSC 0,1X -SDS 0,1 % à 55°C pour HIVSET et polV2 (37°C pour SK38-39). La coloration des membranes et la révélation sont réalisées comme indiqué par le fabriquant (Boehringer, réf 1093657).
   Pour éviter toute contamination, la préparation des réactifs aliquotés, l'extraction de l'ADN des échantillons, la réaction de PCR à proprement parler, l'analyse des produits d'amplification, sont réalisées dans 4 locaux différents, géographiquement distants possédant leur propre matériel.

### 2) Résultats

**2.1) Amplification par les amorces mutées :** L'intensité du signal obtenu au Southern-blot est graduée de 0 à 5 ( 0 = absence de bande, 5 = bande à la dilution 10⁻⁴ du produit d'amplification). L'amorce de référence donne un signal égal à 4. Les résultats de l'amplification ne sont affectés que lorsque la mutation intéresse l'un des deux derniers nucléotides de la séquence de l'amorce à son extrémité 3'(figure 3). Ainsi, si la cytosine en 3' de l'amorce 1-F est remplacée par une guanine ou une adénine, le signal est égal à 1 ou nul (substitution d'une base pyrimidique par une base purique). Si elle est remplacée par une thymidine, le signal est égal à 2 (substitution d'une base pyrimidique par une base pyrimidique). Lorsque la thymidine de l'avant dernière position de la séquence est remplacée par une adénine, le signal est nul. Lorsqu'elle est remplacée par une guanine, il est égal à 2, lorsqu'elle est remplacée par une cytosine, il est égal à 3. Toute substitution de base concernant un nucléotide situé en amont de l'avant dernière position conduit à obtenir un signal égal à 3 ou 4.
**2.2) Comparaison HIVSET et amorces classiques dans la gp 120 :** On a comparé à l'électrophorèse et en Southern Blot, l'amplification de 17 souches de VIH par les "*amorces en escalier"* selon la présente invention à celles réalisées avec des couples simples d'amorces, constitués des amorces standards (uni-moléculaires) de 25 nucléotides (1-F et 2-F), et de 20 nucléotides (1K et 2K)(figure 2). Les résultats sont rapportés sur le tableau 2 : 17 souches/17 sont amplifiées par HIVSET contre 15/17 par les amorces de 25 nucléotides et 12/17 par celles de 20 nucléotides. Le génome du VIH a été recherché dans lymphocytes de 10 de ces 17 patients. Il est retrouvé 10 fois / 10 par HIVSET contre 9 fois / 10 et 4 fois / 10 par les amorces de 25 et 20 nucléotides respectivement.
**2.3) Comparaison de HIVSET aux amorces des régions conservées polV2 et SK38-39** : Sur ces 17 souches, les résultats sont de 17 amplifications / 17 pour polV2, et de 16 / 17 pour SK38-39. Le génome du VIH a été mis en évidence 10 fois dans les lymphocytes de 10 de ces malades par SK38-39 et par polV2 (Tableau 2 ci-après). 55 autres souches virales provenant de 55 malades différents (Figure 4) ont été amplifiées par les amorces HIVSET et par les amorces polV2 et SK38-39 : 55 souches / 55 ont été amplifiées par HIVSET et SK38-39, et 54 : 55 par polV2. Il faut noter qu'une souche donne avec HIVSET une bande très faible à l'hybridation et de taille supérieure à 437 pb. On a pu amplifier les séquences du VIH, par les 3 systèmes, à partir des lymphocytes de 12 malades / 12 étudiés dans ce groupe. Au total sur 72 souches 71 ont été amplifiées par SK et polV2 et 72 / 72 par HIVSET.
   Tous les discordants ont été vérifiés 2 fois (Réextraction de l'ADN). Les témoins négatifs des diverses séries d'extraction et d'amplification sont toujours restés négatifs. 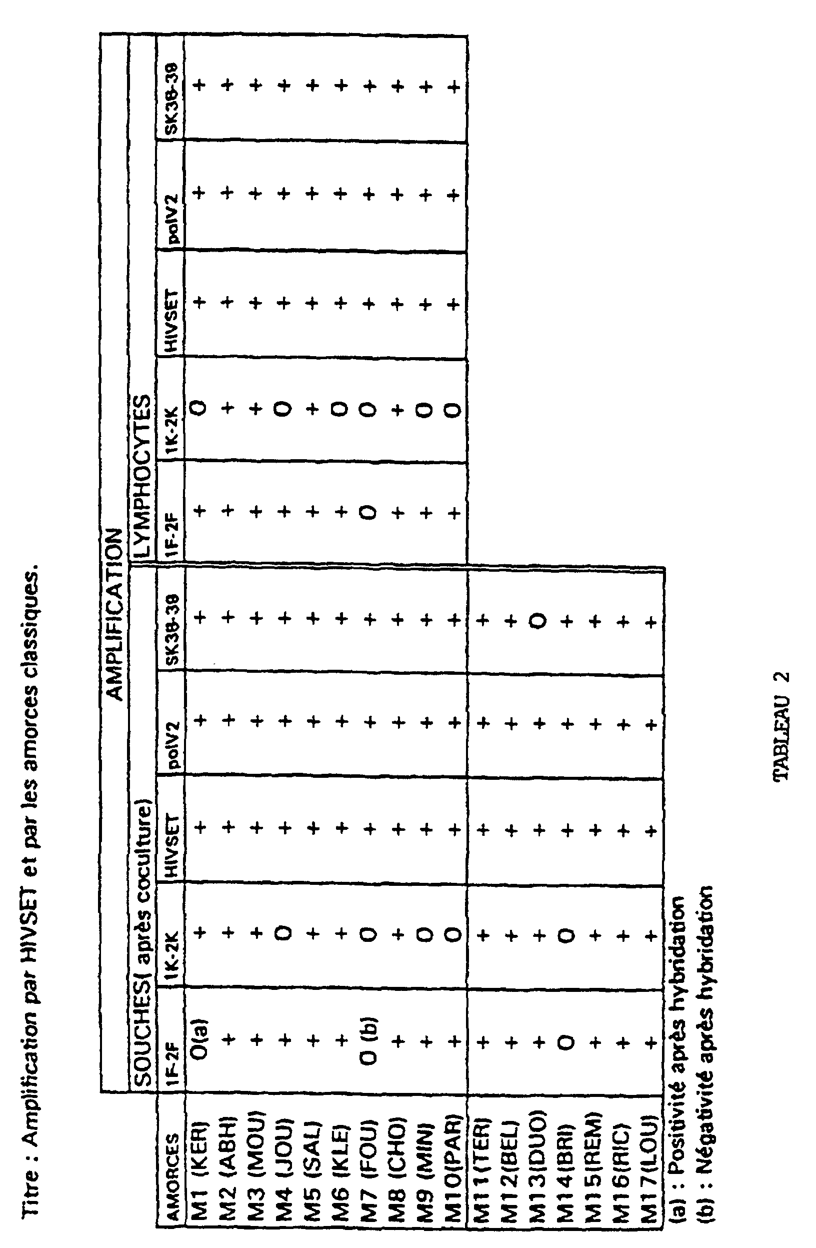
**2.4) Comparaison des séquences amorces HIVSET à celles de la banque génomique :** 210 séquences de VIH référencées dans la banque Embl ont été comparées aux séquences des amorces par le programme Blastn. On constate qu'il existe de 83 à 100 % d'homologie entre régions correspondantes. La projection de l'amorce 1-A sur la région génomique homologue a permis de retenir 33 variants différents représentant l'ensemble des mutations rencontrées dans cette portion de génome.
   L'analyse graphique (figure 5) de la position de ces mutations fait ressortir que les variations ne portent jamais sur plus de 3 bases consécutives, les mutations les plus fréquentes sont ponctuelles. Une séquence de 30 bases ne contient jamais plus de 3 mutations. Etant donné qu'une mutation portant sur une base n'affecte le rendement de l'amplification que si elle se situe à moins de 3 bases de l'extrémité 3' de l'amorce, on peut conclure que, quel que soit le génome viral, plus de la moitié des espèces moléculaires du mélange seront capables d'amorcer l'amplification.
   La même analyse réalisée pour l'amorce 2-A aboutit aux mêmes constatations (83 à 100 % d'homologie, résultats non montrés).

Les amorces en escalier utilisées ci-dessus ont permis d'amorcer de façon constante des régions du VIH comportant jusqu'à 17 % de divergences ponctuelles de séquences.
Dans des conditions d'amorçage peu stringentes, la comparaison des "amorces escalier" à des amorces uni-moléculaires de la même région (1K, 2K), (résultats non montrés) a permis de constater la quasi-absence d'amorçage non spécifique avec les "amorces *en escalier".* La crainte était de voir apparaître de nombreuses bandes non spécifiques dues à la multiplicité des terminaisons 3'. Chaque espèce moléculaire représente seulement le 11ème de la molarité nécessaire pour amplifier efficacement, ce fait explique vraisemblablement ce constat. Les amorces en escalier peuvent donc être utilisées comme moyen supplémentaire pour éviter les amorçages non spécifiques.

### BIBLIOGRAPHIE

1-Altschul, Stephen F., Warren Gish, Webb Miller, Eugene W., Myers, and David J. Lipman. Basic local alignment search tool, J. Mol. Biol. 1990, 215 : 403-410.
2 - Barun K.D., Srinivasan A. Multiple primer pairs for detection of HTLV-I by PCR. Nucleic Acids Research 1989, 17 : 2142.
3 - Busch M.P., Henrard D.R., Hewlett I.K., Mehaffey W.F., Epstein J.S., Allain J.P., Lee T.Z., Mosley J. W. and the transfusion safety study group. Poor sensitivity, Specifity and reproducibility of detection of HIV-1 DNA in serum by polymerase chain reaction. Journal of Acquired Immunodeficiency Syndromes 1992, 5 : 872-877.
4 - Candotti D., Jung M., Kerouedan D., Rosenheim M., Gentilini M., M'Pele P;, Huraux J.M., Agut H. Genetic variability affects the detection of HIV by polymerase chain reaction. AIDS 1991, 5 : 1003-1007.
5 - Chamberlain J.S., Gibbs R.A., Ranier J.E, Caskey C.T. (1990). In : Innis M.A., Gelfand D.H., Snmnisky J.J., White T.J. eds. PCR Protocols : A guide to methods and applications. Academic Press Inc., San Diego, pp.272-281.
6 - Coutlée F., Viscidi R.P., Sainto Antoine P., Kessous A., Yolken R.H. The polymerase chain reaction : a new tool for the understanding and diagnosis of HIV-1 infection at the molecular level. Molecular and Cellular Probes 1991, 5 : 241-259.
7 - David D., Ho M.D., Tarsem Moudgil M.S. and Masud Alam M.S. Quantitation of human immunodeficiency virus type 1 in the blood of infected persons. N. EngL J. Med. 1989; 32:1621-1625.
8 - Folks T.M. et al. A chronically HIV infected T cell clone that constitutively express HIV. J. Exp. Med. 1986, 164 : 280-290.
9 - Griffais R., André P.M. and Thibon M. Synthesis of digoxigenin Labelled DNA probe by polymerase chain reaction, application to Epstein-Barr virus and chlamydia trachomatis. Research in Virology 1990, 14 : 331-335.
10 - Griffais R., André P. M., Thibon M. K-tuple frequency in the human genome and polymerase chain reaction. Nucleic Acids Research 1991, 19 : 3887-3891.
11 - Jung M., Candotti D., Huraux J.M., Agut H. Polymerase chain reaction in human immunodeficiency virus diagnosis : principles, parameters, applications and pitfalls. Bull. Inst. Pasteur 1992, 90 : 31-43.
12 - Knoth K., Roberds S., Poteet C., Tamkun M. Highly degenerate Inosine-containing primers specifically amplify rare cDNA using the polymerase chain reaction. Nucleic Acids Research 1988; 16 : 10932.
13 - Kwok S., Kellog D.E., Mckinney N., Sasic D., Levenson C., Sninsky J.J. Effects of primer template mismatches on the polymerase chain reaction : human immunodeficiency virus type 1 model studies. Nucleic Acids. Res. 1990, 4:999-1005.
14 - Langley M.J., Bennett S.E, Mosbaugh D.W. , Characterization of the 5' to 3' exonuclease associated with thermus aquaticus DNA polymerase. Nucleic Acids Research, 1990, 18:7317-7322.
15 - Levy J.A. Pathogenesis of human immunodeficiency virus infection. Microbiological Reviews, 1993, 57 : 183-289.
16 - Lewis D.E., Gibbs R.A. Detection and significance of HIV sequences in HIV infection. In : Melnick J.L. (ed) : Prog. Med. Virol. Basel, Karger 1993, 4 :, 19-47.
17 - Linz V., Rubsamen-Waigmann H., Roesch R., Seliger H. Inosine substituted primers prevent false negative PCR results due to 3' mismatches. Amplification 1990, 4 : 14-15.
18 - Locke M. , Mach B. Identification of HIV infected seronegative individuals by a direct diagnostic test based on hybridisation to amplified viral DNA. Lancet 1988, 11: 418-421.
19 - Meyerhans A., Cheynier R., Albert J., Seth M., Kwok S., Sininsky J., Morfeldt-Manson L, Asjö B., Wain-Hobson. Temporal fluctuations in HIV quasispecies in vivo are not reflected by sequental HIV isolations. Cell, 1989, 58 : 901-910.
20 - Ou C.Y., Kwok S., Mitchell S.W., Mack D.H., Sninsky J.J., Krebs J.W., Feorino P., Warfield D., Schochetman S. DNA amplification for direct detection of HIV-1 in DNA of peripheral. Blood Mononuclear Cells. Science 1988, 239: 295-297.
21 - Saiki R.K., Gelfand D.H., Stoffel. Primer-directed enzymatic amplification of DNAwith a thermostable DNA polymerase. Science 1988, 27: 6008-6013.
22 - Tindall K.R. and Kunkel T.A. Fidelity of DNA synthesis by the Thermus Aquaticus DNA Polymerase. Biochemistry, 1988, 27: 6008-6013.
23 - Vaishnav Y.N. and Wong-Staal F. The biochemistry of AIDS. Annu. Rev. Biochem. 1991: 60, 577-630.
24 - Zazzi M., Romano L., Brasini A., Valensin P.E. Simultaneous amplification of multiple HIV-DNA sequences from clinical specimens by using Nested-primer polymerase chain reaction. AIDS Research and human retroviruses, 1993, 9 : 315-320.
25 - EP-A-0 393 743

## Revendications

1. Procédé d'amplification enzymatique in vitro d'un fragment d'ADN dans lequel on utilise deux amorces respectivement amont et aval dont au moins une est une amorce dite "*en escalier"* qui consiste en un mélange d'oligonucléotides, les oligonucléotides constituant le mélange étant de tailles différentes, de séquences complémentaires à la même région en amont ou en aval du dit fragment à amplifier et chaque oligonucléotide ayant, à partir de son extrémité 5', dans le sens 5'→3', la même séquence que les oligonucléotides plus petits du mélange; **caractérisé en ce que** les oligonucléotides constituant le mélange sont en quantités équimolaires.

2. Procédé d'amplification enzymatique in vitro d'un fragment d'ADN selon la revendication 1, dans lequel on utilise deux amorces en escalier respectivement amont et aval, qui consistent chacune en un mélange d'oligonucléotides, les oligonucléotides constituant chaque mélange amont ou aval étant en quantités équimolaires, de tailles différentes, de séquences complémentaires à la même région respectivement en amont ou en aval du dit fragment à amplifier et chaque oligonucléotide ayant, à partir de son extrémité 5', dans le sens 5'→3', la même séquence que les oligonucléotides plus petits du même mélange.

3. Procédé selon la revendication 1 ou 2, **caractérisé en ce que** une amorce en escalier comporte de 5 à 20 oligonucléotides.

4. Procédé selon la revendication 1, 2 ou 3, **caractérisé en ce que** la différence de taille entre chaque oligonucléotide et celui immédiatement plus petit ou plus grand d'une même amorce est de 1 à 4 nucléotides.

5. Procédé selon l'une des revendications 1 à 4, **caractérisé en ce qu'**on utilise deux amorces en escalier respectivement amont et aval, dans lesquelles le nombre et les tailles des oligonucléotides respectifs des amorces amont et aval sont identiques.

6. Procédé selon l'une des revendications 1 à 5, **caractérisé en ce que**, dans l'amorce en escalier, les différences de tailles entre chaque oligonucléotide et l'oligonucléotide immédiatement plus grand ou plus petit sont identiques.

7. Procédé selon l'une des revendications 1 à 6, **caractérisé en ce que** la taille des oligonucléotides dans une amorce en escalier est de 15 à 40, de préférence 20 à 30 nucléotides.

8. Procédé selon l'une des revendications 1 à 7, **caractérisé en ce que** la ou les amorce(s) est(sont) constituée(s) d'oligonucléotides complémentaires aux régions les mieux conservées de génomes constituant le fragment à amplifier, de préférence des régions qui ne subissent que des mutations ponctuelles.

9. Procédé de diagnostic impliquant la détection in vitro d'un fragment d'ADN dans lequel on utilise un procédé d'amplification enzymatique in vitro du dit fragment d'ADN à détecter selon l'une des revendications 1 à 8.

10. Procédé de diagnostic de maladies virales impliquant la détection d'un virus tel que le virus de l'hépatite C, le virus du SIDA, les virus d'herpès, les virus entériques selon la revendication 9.

11. Kit de diagnostic pour la mise en oeuvre d'un procédé de diagnostic selon la revendication 9 ou 10, comportant des amorces d'amplification enzymatique in vitro d'un fragment d'ADN à détecter consistant en des amorces en escalier **caractérisé en ce que** lesdites amorces consistent en un mélange d'oligonucléotides tels que définis dans l'une des revendications 1 à 8.

## Patentansprüche

1. Verfahren zur enzymatischen Amplifikation eines DNS-Fragmentes in vitro, in welchem man zwei Primer stromaufwärts bzw. stromabwärts verwendet, von denen mindestens einer ein sogenannter "Treppenprimer" ist, der aus einer Mischung von Oligonukleotiden besteht, wobei die Oligonukleotide, welche die Mischung ausmachen, verschiedene Größen mit Sequenzen aufweisen, die komplementär sind zu der gleichen Region stromaufwärts oder stromabwärts bezüglich des zu amplifizierenden Fragments, und jedes Oligonukleotid ausgehend von seinem 5'-Ende in der Richtung 5'→3' die gleiche Sequenz aufweist wie die kleineren Oligonukleotide der Mischung, **dadurch gekennzeichnet, daß** die Oligonukleotide, welche die Mischung ausmachen, in äquimolaren Mengen vorliegen.

2. Verfahren zur enzymatischen Amplifikation eines DNS-Fragments in vitro gemäß Anspruch 1, in welchem man zwei Treppenprimer stromaufwärts bzw. stromabwärts verwendet, die jeweils aus einer Mischung von Oligonukleotiden bestehen, wobei die Oligonukleotide, welche jede Mischung stromaufwärts oder stromabwärts ausmachen, in äquimolaren Mengen mit unterschiedlichen Größen mit Sequenzen vorliegen, die komplementär sind zu der gleichen Region stromaufwärts oder stromabwärts bezüglich des zu amplifizierenden Fragments, und jedes Oligonukleotid ausgehend von seinem 5'-Ende in Richtung 5''→3' die gleiche Sequenz aufweist wie die kleineren Oligonukleotide derselben Mischung.

3. Verfahren nach Anspruch 1 oder 2, **dadurch gekennzeichnet, daß** ein Treppenprimer 5 bis 20 Oligonukleotide umfaßt.

4. Verfahren nach Anspruch 1, 2 oder 3, **dadurch gekennzeichnet, daß** der Unterschied der Größe zwischen jedem Oligonukleotid und dem unmittelbar kleineren oder größeren desselben Primers 1 bis 4 Nukleotide beträgt.

5. Verfahren nach einem der Ansprüche 1 bis 4, **dadurch gekennzeichnet, daß** man zwei Treppenprimer stromaufwärts bzw. stromabwärts verwendet, in welchen die Zahl und die Größen der jeweiligen Oligonukleotide des stromaufwärtigen bzw. stromabwärtigen Primers identisch sind.

6. Verfahren nach einem der Ansprüche 1 bis 5, **dadurch gekennzeichnet, daß** in dem Treppenprimer die Unterschiede der Größen zwischen jedem Oligonukleotid und dem unmittelbar größeren oder kleineren Oligonukleotid identisch sind.

7. Verfahren nach einem der Ansprüche 1 bis 6, **dadurch gekennzeichnet, daß** die Größe der Oligonukleotide in einem Treppenprimer 15 bis 40, vorzugsweise 20 bis 30 Nukleotide beträgt.

8. Verfahren nach einem der Ansprüche 1 bis 7, **dadurch gekennzeichnet, daß** der oder die Primer aus Oligonukleotiden besteht bzw. bestehen, die komplementär sind zu den am meisten konservierten Regionen von Genomen, welche das zu amplifizierende Fragment ausmachen, vorzugsweise Regionen, die nur Punktmutationen unterliegen.

9. Diagnostisches Verfahren, welches den In-vitro-Nachweis eines DNS-Fragments beinhaltet, in welchem man ein Verfahren zur enzymatischen Amplifikation des nachzuweisenden DNS-Fragments in vitro gemäß einem der Ansprüche 1 bis 8 verwendet.

10. Diagnostisches Verfahren für virale Erkrankungen, welches den Nachweis eines Virus, wie des Hepatitis C-Virus, des AIDS-Virus, des Herpes-Virus, enterischer Viren, gemäß Anspruch 9 beinhaltet.

11. Diagnostischer Testsatz für die Durchführung eines diagnostischen Verfahrens nach Anspruch 9 oder 10, welcher Primer der enzymatischen Amplifikation eines nachzuweisenden DNS-Fragments in vitro umfaßt, welche aus Treppenprimern bestehen, **dadurch gekennzeichnet, daß** die Primer aus einer Mischung von Oligonukleotiden bestehen, wie in einem der Ansprüche 1 bis 8 definiert.

## Claims

1. Method for enzymatically amplifying a DNA fragment in vitro, in which use is made of two primers, an upstream and a downstream primer, respectively, at least one of which is a "step" primer which consists of a set of oligonucleotides, the oligonucleotides making up the set being different in size and having sequences complementary to the same region upstream or downstream of said fragment to be amplified, and each oligonucleotide having, from its 5' end, in the 5' → 3' direction, the same sequence as the shorter oligonucleotides of the set, **characterized in that** the oligonucleotides making up the set are in equimolar amounts.

2. Method for enzymatically amplifying a DNA fragment in vitro, according to Claim 1, in which use is made of two step primers, an upstream and a downstream primer, respectively, which each consist of a set of oligonucleotides, the oligonucleotides making up each upstream or downstream set being in equimolar amounts and of different sizes and having sequences complementary to the same region upstream or downstream, respectively, of said fragment to be amplified, and each oligonucleotide having, from its 5' end, in the 5' → 3' direction, the same sequence as the shorter oligonucleotides of the same set.

3. Method according to Claim 1 or 2, **characterized in that** a step primer comprises from 5 to 20 oligonucleotides.

4. Method according to Claim 1, 2 or 3, **characterized in that** the difference in size between each oligonucleotide and the one immediately shorter or longer, of the same primer, is 1 to 4 nucleotides.

5. Method according to one of Claims 1 to 4, **characterized in that** use is made of two step primers, an upstream and a downstream primer, respectively, in which the number and sizes of the respective oligonucleotides of the upstream and downstream primers are identical.

6. Method according to one of Claims 1 to 5, **characterized in that**, in the step primer, the differences in sizes between each oligonucleotide and the oligonucleotide immediately longer or shorter are identical.

7. Method according to one of Claims 1 to 6, **characterized in that** the size of the oligonucleotides in a step primer is 15 to 40, preferably 20 to 30, nucleotides.

8. Method according to one of Claims 1 to 7, **characterized in that** the primer(s) consist(s) of oligonucleotides complementary to the most well-conserved regions of the genomes making up the fragment to be amplified, preferably regions which undergo only point mutations.

9. Diagnostic method involving the detection of a DNA fragment in vitro, in which use is made of a method for enzymatically amplifying said DNA fragment to be detected, in vitro, according to one of Claims 1 to 8.

10. Method for diagnosing viral diseases, involving the detection of a virus, such as the hepatitis C virus, the AIDS virus, herpesviruses or enteric viruses, according to Claim 9.

11. Diagnostic kit for carrying out a diagnostic method according to Claim 9 or 10, comprising primers for enzymatically amplifying a DNA fragment to be detected, in vitro, consisting of step primers, **characterized in that** said primers consist of a set of oligonucleotides as defined in one of Claims 1 to 8.
